# EUROPEAN PATENT APPLICATION

(11) **EP 4 725 954 A1**
(43) Date of publication of application: **15.04.2026**
(21) Application number: 24830326.5
(22) Date of filing: 24.05.2024
(51) Int. Cl.: C07K 7/06, C07K 7/08, A61K 38/08, A61P 39/06, A61P 17/18, A23L 33/18

(54) **MODIFIED PEPTIDES SUCH AS POLYPEPTIDES LA-19 AND TM-19 HAVING ANTI-AGING AND REPAIRING EFFECTS AND USE THEREOF**

(30) Priority: 26.06.2023 WO PCT/CN2023/102520
(71) Applicant: Anhui Dexin Huahai Biological Technology Co. Ltd., Maanshan, Anhui 243099 (CN)
(72) Inventor: KANG, Mengshi, Maanshan, Anhui 243099 (CN); WANG, Yunfan, Maanshan, Anhui 243099 (CN)
(74) Representative: Prüfer & Partner mbB Patentanwälte · Rechtsanwälte
(86) International application number: PCT/CN2024/095256
(87) International publication number: WO 2025/001678

(57) **Abstract**

The present application relates to the field of molecular biology, in particular to polypeptides and the use thereof, a composition for relieving or treating ultraviolet damage, oxidation, inflammation and aging and the use thereof.

## Description

### TECHNICAL FIELD

The present application relates to the field of cosmetics or pharmaceuticals, and in particular to a polypeptide and use thereof, and a composition for relieving or treating ultraviolet damage, oxidation, inflammation and aging and use thereof.

### BACKGROUND

With age, facial skin is prone to aging, characterized by increased wrinkles.. As the organ with the most contact with the external environment, skin is also gradually aging like other organs, leading to the weakening or loss of function, which not only affects appearance but also increases the incidence of various skin diseases. Skin aging refers to the senile degeneration of skin function caused by the stimulation from internal and external environment such that the perceptual ability and regenerative capacity of skin are reduced, thus making it unable to promptly repair or reduce the damage caused by internal and external environment to the body. Exogenous skin aging is mainly caused by environmental factors such as ultraviolet (UV) radiation, sun exposure, and exposure to harmful chemicals. UV-caused photoaging not only leads to skin laxity, roughness, light-yellow or grayish-yellow skin discoloration, telangiectasia, and formation of pigmented spots, but also causes degradation of skin collagen and accumulation of abnormal elastin fibers in severe cases, thereby inducing acute skin inflammatory reactions to trigger skin carcinogenesis.

In recent years, the application of polypeptide raw materials has become a key focus in the research and development of cosmetic efficacy products. Due to good tolerance, high safety, and significant efficacy, these raw materials have broad application prospects in skin anti-aging applications. Anti-aging polypeptides in the prior art include the following: acetyl hexapeptide-8 whose mechanism is the same as botulinum toxin and advantage is relatively safe and to have significant clinical and practical efficacy; palmitoyl pentapeptide, as one of the earliest signal peptides applied to skin-care products, can effectively improve skin roughness, reduce the number of fine wrinkles, decrease the depth and area of wrinkles, and is featured by being mild and non-irritating; palmitoyl tetrapeptide-7 is known for its anti-inflammatory effect, capable of inhibiting inflammatory mediators, reducing white blood cell count, alleviating skin inflammatory injury, and is added in many skin-care products.

Therefore, it is necessary to discover more polypeptides that are safe and exhibit significant anti-aging efficacy to further expand the diversity of polypeptides with anti-aging efficacy, thus providing more options for the development of cosmetics, health-care products, functional foods, and pharmaceuticals.

It needs to be noted that the methods described in this section are not necessarily those that have been conceived or adopted previously. Unless otherwise specified, it should not be assumed that any of the methods described in this section is considered as the prior art merely by virtue of its inclusion in this section. Similarly, unless otherwise specified, the problems mentioned in this section shall not be considered as having been widely recognized in any prior art.

### SUMMARY

Based on this, to seek for novel polypeptides with high safety and anti-aging activity, the present application provides a polypeptide, including at least one amino acid sequence having at least 80%, 90%, 95%, or 99% identity to an amino acid sequence as shown in any one of SEQ ID NOs: 1-10.

The novel polypeptides disclosed in the present application have multiple anti-aging related activity such as anti-inflammatory, antioxidant, UV-protective, and skin-protective effects and thus, are of great significance for the development of cosmetics, health-care products, food and pharmaceuticals that have related functions. Specifically, the polypeptides can resist UV damage to the skin and improve the body's antioxidant and anti-inflammatory abilities. Meanwhile, the polypeptides also have good safety, which is not only free of affecting the growth of skin keratinocytes, but also is capable of promoting keratinocyte proliferation and skin keratin regeneration.

Furthermore, compared to the antioxidant ingredients of retinol and the anti-inflammatory polypeptide palmitoyl tetrapeptide-7 relatively widely used in the prior art, the polypeptides disclosed in the present application have better UV radiation resistance, oxidation resistance, anti-inflammatory effect, and other anti-aging related activity.

According to one embodiment of the present application, provided is a composition for alleviating or treating UV damage, oxidation, inflammation, or aging, including: a polypeptide having an amino acid sequence as shown in any one of SEQ ID NOs: 1-10; and a pharmaceutically, nutritionally, or physiologically acceptable excipient.

According to one embodiment of the present application, provided is use of the polypeptide of the present application in the preparation of an anti-UV, antioxidant, anti-inflammatory, or anti-aging product.

According to one embodiment of the present application, provided is use of a polypeptide having an amino acid sequence of TKAAA in the preparation of an anti-UV, antioxidant, anti-inflammatory, or skin anti-aging product.

According to one embodiment of the present application, provided is use of the polypeptide of the present application and the composition of the present application in alleviating or treating UV damage, oxidation, inflammation, or aging.

According to one embodiment of the present application, provided is use of the polypeptide having an amino acid sequence of TKAAA in alleviating or treating UV damage, oxidation, inflammation, or skin aging.

It should be understood that the content described in this section is neither intended to identify key or important features of the embodiments of the present application, nor construed as limiting the scope of the present application. Other features of the present application will become easy to understand through the following description.

### BRIEF DESCRIPTION OF THE DRAWINGS

The drawings exemplarily show the embodiments and constitute a part of the description, serving to explain the exemplary implementation of the embodiments together with the text description of the description. The shown embodiments are for illustrative purposes only, but are not construed as limiting the scope of the claims. The same reference numerals in all drawings refer to similar but not necessarily identical elements.

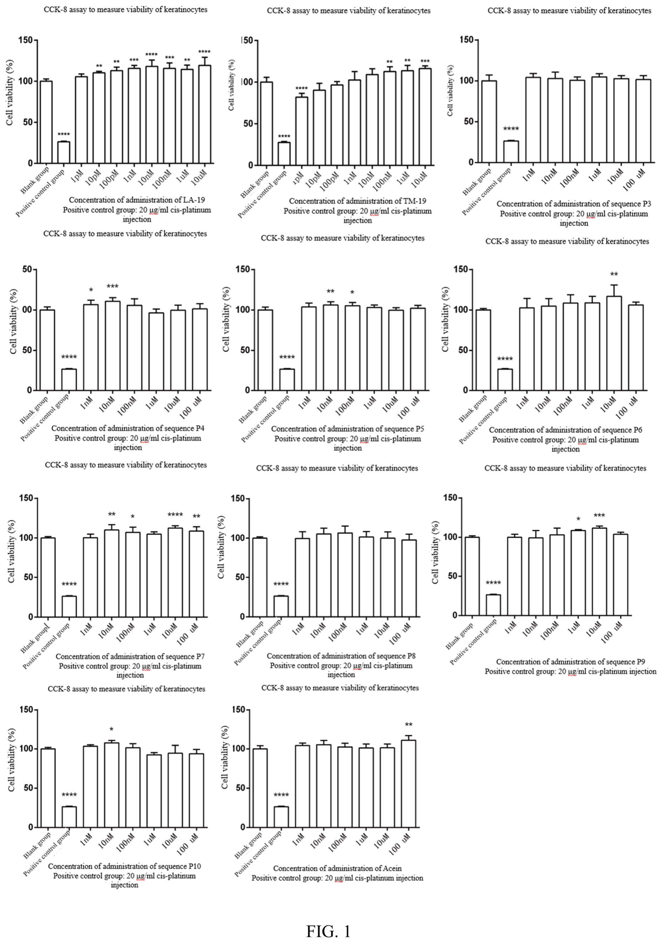

FIG. 1 shows cytotoxicity test results of polypeptides on keratinocytes in Example 1, wherein, from left to right are the test results for polypeptides LA-19, TM-19, P3-10, and Acein. The results are expressed as Mean ± SEM, compared with the blank group, *P<0.05 denotes a significant difference, **P<0.01, ***P<0.001, and ****P<0.0001 denote very significant differences.

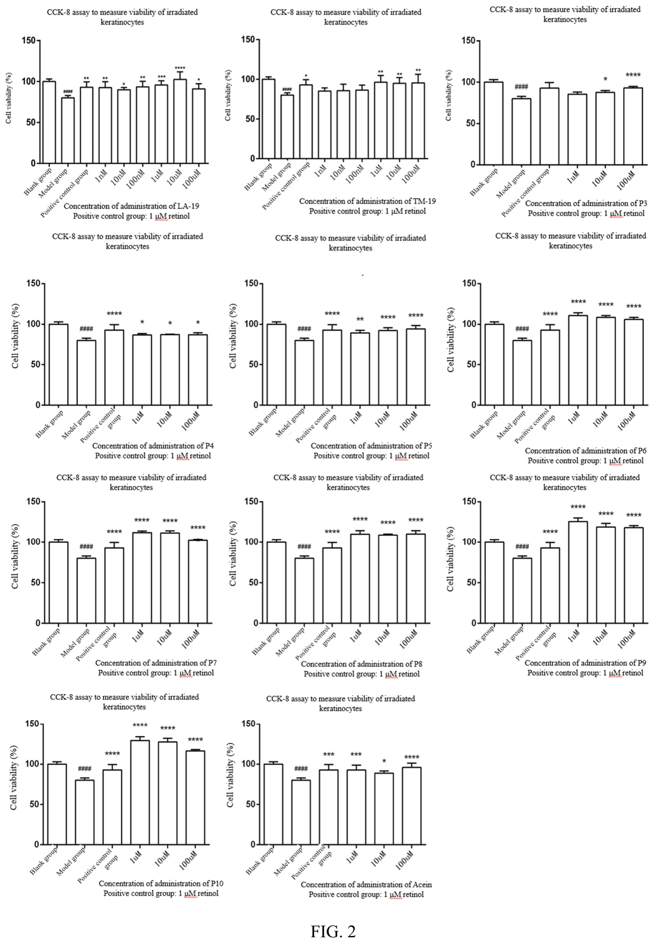

FIG. 2 shows anti-UV activity test results of the polypeptides on keratinocytes in Example 2, wherein, from left to right are the test results for polypeptides LA-19, TM-19, P3-10, and Acein. The results are expressed as Mean ± SEM, compared with the blank group, in model group, ####P<0.0001 denotes a very significant difference, indicating the successful establishment of a UV damage model; compared with the model group, *P<0.05 denotes a significant difference, **P<0.01, ***P<0.001, and ****P<0.0001 denote very significant differences.

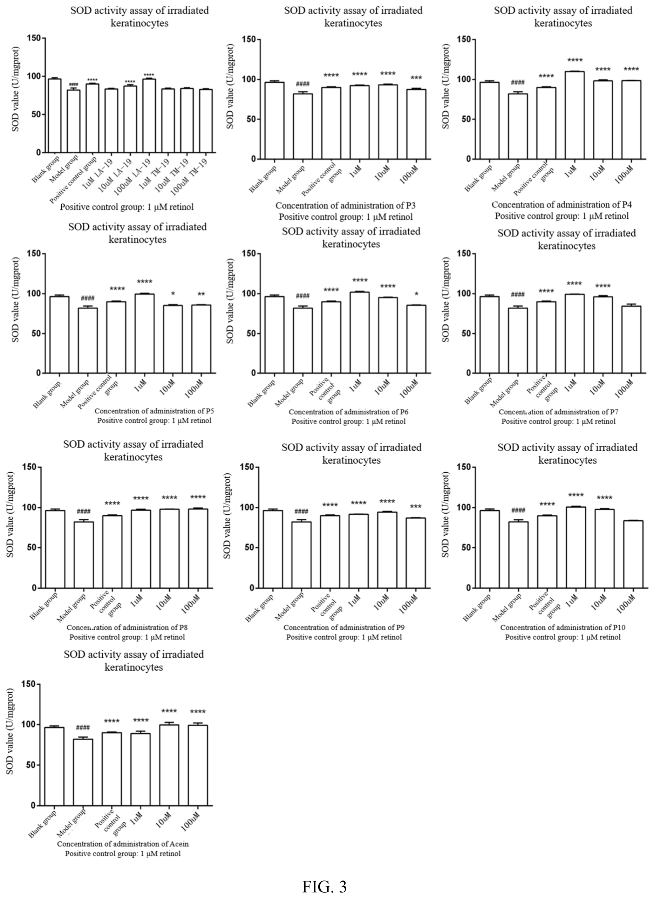

FIG. 3 shows antioxidant activity test results of the polypeptides on keratinocytes in Example 3. The activity of superoxide dismutase (SOD) in cells represents the antioxidant capacity. The results are expressed as Mean ± SEM, compared with the blank group, in model group, #P<0.05 denotes a significant difference, ####P<0.0001 denotes a very significant difference, indicating the successful establishment of the UV-induced oxidative damage model; compared with the model group, *P<0.05 denotes a significant difference, **P<0.01, ***P<0.001, and ****P<0.0001 denote very significant differences.

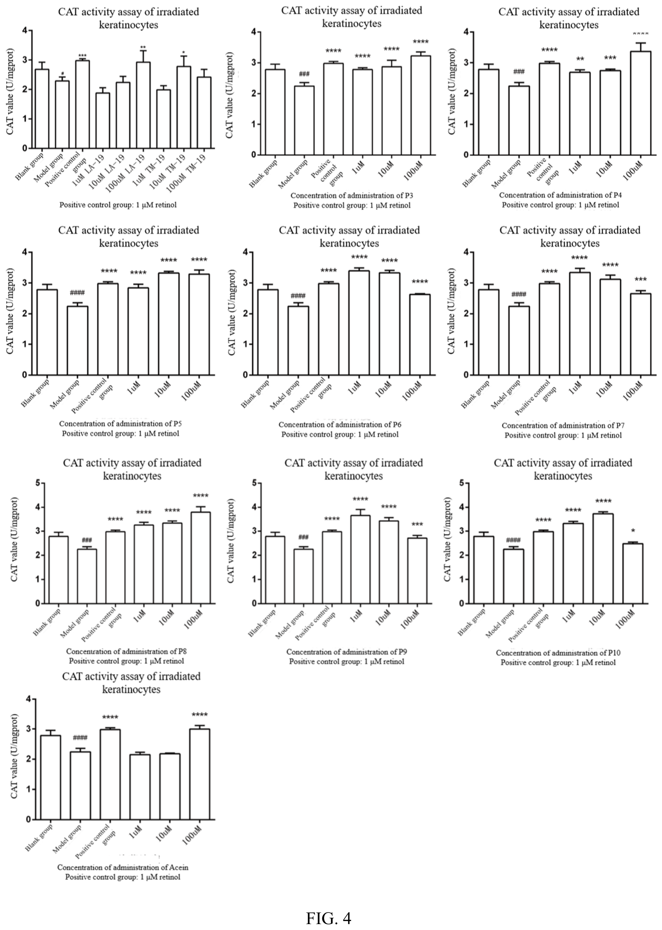

FIG. 4 shows antioxidant activity test results of the polypeptides on keratinocytes in Example 3. The catalase (CAT) in cells represents the antioxidant capacity. The results are expressed as Mean ± SEM, compared with the blank group, in model group, #P<0.05 denotes a significant difference, ####P<0.0001 denotes a very significant difference, indicating the successful establishment of the UV-induced oxidative damage model; compared with the model group, *P<0.05 denotes a significant difference, **P<0.01, ***P<0.001, and ****P<0.0001 denote very significant differences.

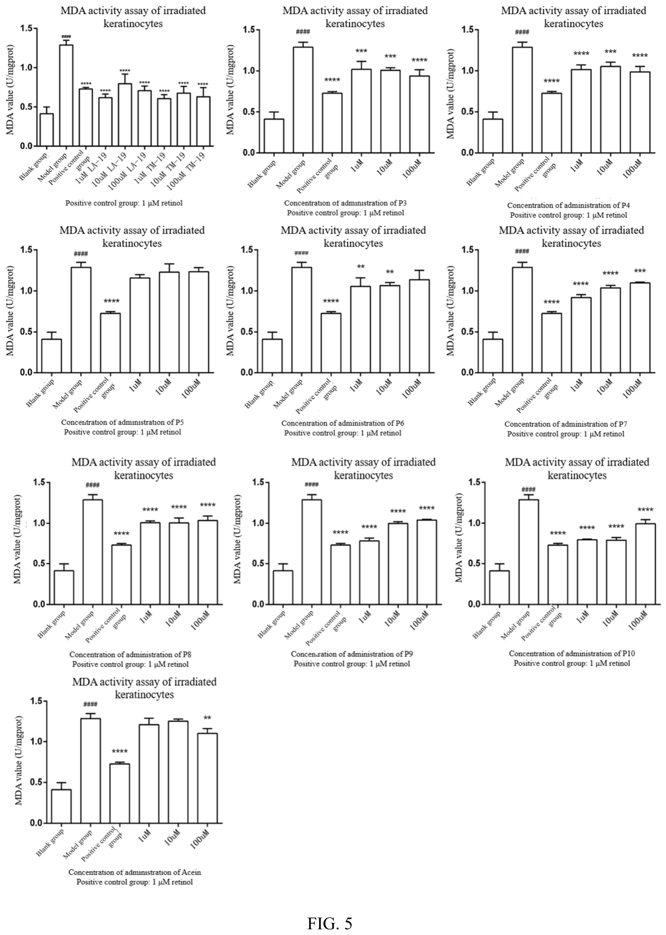

FIG. 5 shows antioxidant activity test results of the polypeptides on keratinocytes in Example 3. The activity of malondialdehyde (MDA) in cells represents the degree of intracellular oxidative damage. The results are expressed as Mean ± SEM, compared with the blank group, in model group, #P<0.05 denotes a significant difference, ####P<0.0001 denotes a very significant difference, indicating the successful establishment of the UV-induced oxidative damage model; compared with the model group, *P<0.05 denotes a significant difference, **P<0.01, ***P<0.001, and ****P<0.0001 denote very significant differences.

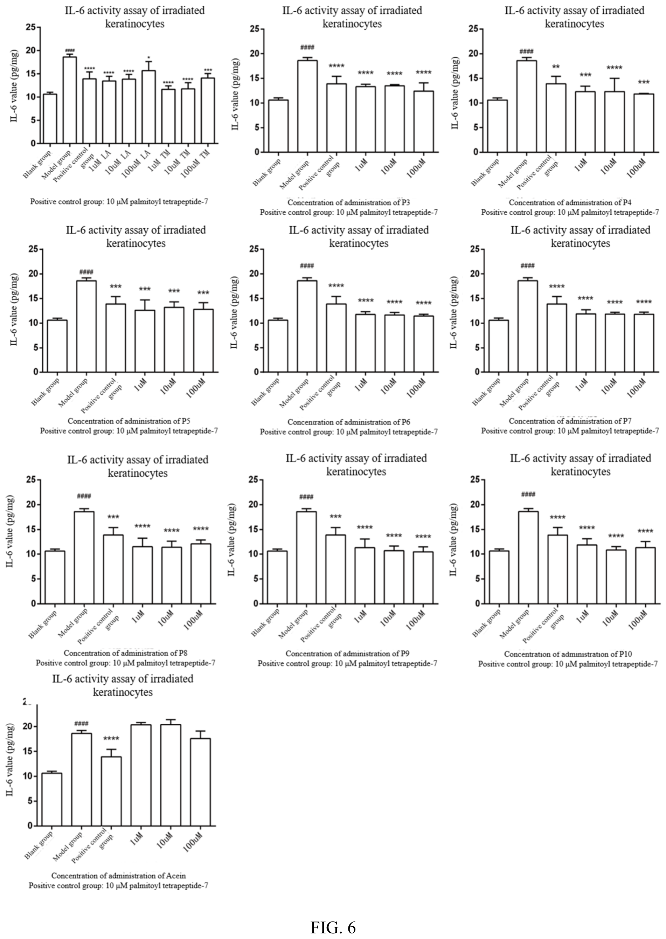

FIG. 6 shows anti-inflammatory activity test results of the polypeptides on keratinocytes in Example 4. The inflammatory factor IL-6 represents the level of inflammation in cells. The results are expressed as Mean ± SEM, compared with the blank group, in model group, ###P<0.001 and ####P<0.0001 denote very significant differences, indicating the successful establishment of the UV-induced cell inflammation model; compared with the model group, *P<0.05 denotes a significant difference, **P<0.01, ***P<0.001, and ****P<0.0001 denote very significant differences.

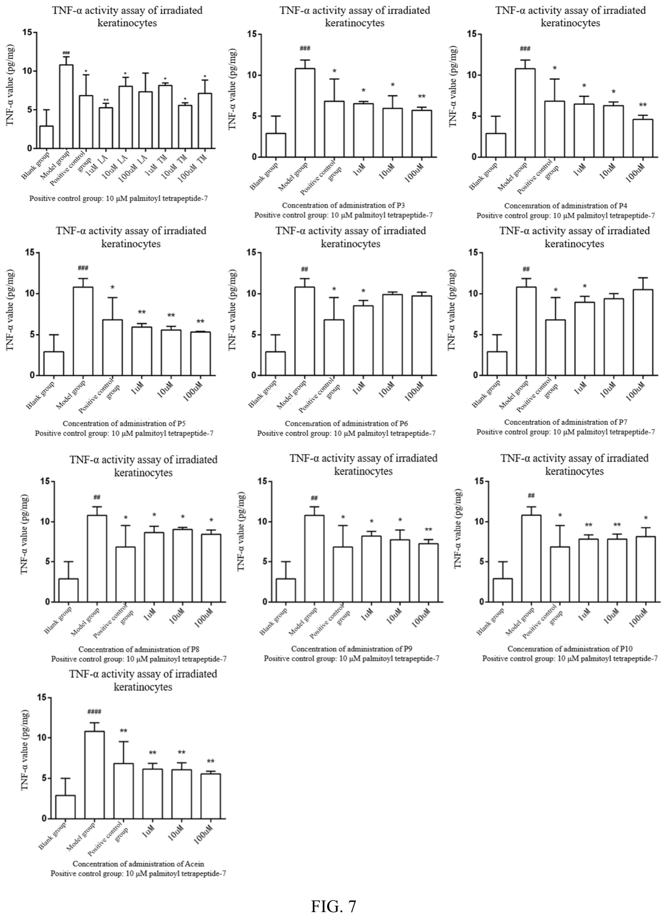

FIG. 7 shows anti-inflammatory activity test results of the polypeptides on keratinocytes in Example 4. The inflammatory factor TNF-α represents the level of inflammation in cells. The results are expressed as Mean ± SEM, compared with the blank group, in model group, ###P<0.001 and ####P<0.0001 denote very significant differences, indicating the successful establishment of the UV-induced cell inflammation model; compared with the model group, *P<0.05 denotes a significant difference, **P<0.01, ***P<0.001, and ****P<0.0001 denote very significant differences.

### DETAILED DESCRIPTION OF THE EMBODIMENTS

Unless otherwise specified or contradicted by the context, the terms or expressions used herein shall be read in conjunction with the entire content of this text and as understood by those skilled in the art. Unless otherwise defined, all technical and scientific terms used herein have the same meaning as commonly understood by those skilled in the art.

As used herein, the "polypeptide" and the "peptide" may be interchangeable and refer to a polymer of amino acids of any length. Therefore, polypeptides, oligopeptides, proteins, antibodies, and enzymes are all included in the definition of polypeptide.

As used herein, a "variant" of a polypeptide sequence is a polypeptide that is different from a reference polypeptide but retains essential properties. A typical variant of a polypeptide differs from another reference polypeptide in amino acid sequence. Generally, the differences are limited; hence, the sequences of the reference polypeptide and the variant are substantially similar and are identical in many regions. Variant polypeptides and reference polypeptides may differ in amino acid sequence by one or more substitutions, additions, deletions in any combination. Any substituted or inserted amino acid residue may or may not be encoded by a genetic code. Variants of polynucleotides or polypeptides may occur naturally, such as allelic variations, or may be unknown naturally occurring variants. Non-naturally occurring variants of polynucleotides and polypeptides may be produced by mutagenesis techniques, direct synthesis, and other recombinant methods known to skilled artisans.

As used herein, the "alleviate" and "treat" and their synonyms refer to the improvement of diseases, disorders, and/or conditions. The "alleviate" and "treat" may be the improvement in at least one measurable physical parameter which is not necessarily recognizable by a patient. The "alleviate" and "treat" may also inhibit the progression of diseases, disorders, and/or conditions physically (e.g., stabilizing recognizable symptoms), physiologically (e.g., stabilizing physical parameters), or both. The "alleviate" and "treat" may also slow down or reverse the development of a disease, a disorder, and/or a condition.

As used herein, the "treat" is intended to cover "prevention". The "prevention" and its synonyms refer to delaying the onset of specific diseases, disorders, and/or conditions or symptoms associated with these diseases, disorders, and/or conditions, or reducing the risk of acquiring these diseases, disorders, and/or conditions.

As used herein, the "pharmaceutically acceptable excipient" refers to a carrier, diluent, or adjuvant used in drug formulation or administration, which is not an essential active ingredient per se and is free of excessive toxicity after administration. Suitable pharmaceutically acceptable excipients are well known to those skilled in the art, including but not limited to, calcium bicarbonate, calcium phosphate, various types of sugars and starches, cellulose derivatives, gelatin, vegetable oils, polyethylene glycol, and surfactants, including, for example, polysorbate 20.

As used herein, the "physiologically acceptable excipient" and the "nutritionally acceptable excipient" refer to a carrier, a diluent, or an adjuvant that neither causes significant irritation to an organism nor eliminates the biological activity and properties of the administered polypeptide.

### Polypeptide

The present application provides a polypeptide comprising at least one amino acid sequence having at least 80%, 90%, 95%, or 99% identity to an amino acid sequence as shown in any one of SEQ ID NOs: 1-10.

In some embodiments, the polypeptide includes a variant sequence of the amino acid sequence as shown in any one of SEQ ID NOs: 1-10; the variant sequence has an identity of not less than 80% to the amino acid sequence as shown in any one of SEQ ID NOS: 1-10, for example, 80%, 81%, 82%, 83%, 84%, 85%, 86%, 87%, 88%, 89%, 90%, 91%, 92%, 93%, 94%, 95%, 96%, 97%, 98%, 99%, or a numerical value within a range consisting of any two of the above points.

In some embodiments, the polypeptide includes an amino acid sequence as shown in any one of SEQ ID NOs: 1-10.

In some embodiments, the polypeptide includes a derivative of the amino acid sequence as shown in any one of SEQ ID NOs: 1-10. In some embodiments, the polypeptide is a derivative of the amino acid sequence as shown in any one of SEQ ID NOs: 1-10. The derivative is obtained by modifying an amino acid side-chain radical, an amino terminal, or a carboxyl terminal of the polypeptide as shown in any one of SEQ ID NOs: 1-10; the modification includes but is not limited to hydroxylation, carboxylation, carbonylation, methylation, acetylation, phosphorylation, esterification, or glycosylation.

In some embodiments, the polypeptide is an amino acid sequence as shown in any one of SEQ ID NOs: 1-10.

In some preferred embodiments, the polypeptide further has terminal modifications including acetylation and/or amidation. By adding modifications onto the polypeptide terminals, the activity of the polypeptide may be further improved and its stability is increased. Terminal modification methods are commonly known methods in the art, and any suitable terminal modification method may be used for the polypeptides disclosed in the present application, which is not limited herein.

### Composition

According to one embodiment of the present application, provided is a composition for alleviating or treating UV damage, oxidation, inflammation, or aging, wherein the composition including: at least one polypeptide having an amino acid sequence as shown in any one of SEQ ID NOs: 1-10; and a pharmaceutically, nutritionally, or physiologically acceptable excipient.

In some embodiments, the composition may be used to alleviate UV damage, oxidation, inflammation, or aging in any part of the body. In some preferred embodiments, the composition is used to alleviate UV damage, oxidation, inflammation, or aging of skin.

Different types of compositions may be selected according to different purposes. In some embodiments, the composition includes a cosmetic composition, health-care product composition, a food composition, or a pharmaceutical composition. In some preferred embodiments, the composition for alleviating UV damage, oxidation, inflammation, or aging of skin is a cosmetic composition.

Suitable dosage forms are well known to those skilled in the art. In some embodiments, the dosage form of the composition includes injections, lyophilized powder injections, tablets, granules, aqueous solutions, oil formulations, creams, gels, emulsions, powders, or aerosols.

Different types of dosage forms may be selected according to different types of compositions. In some preferred embodiments, the optional dosage forms for the cosmetic composition include but are not limited to aqueous solutions, oil formulations, gels, creams, emulsions, powders, or aerosols. In some preferred embodiments, the optional dosage forms for the pharmaceutical composition or health-care product composition include but are not limited to injections, lyophilized powder injections, tablets, or granules.

Suitable excipients are well known to those skilled in the art, for example: liquid, gels, or solid carriers; aqueous vehicles; non-aqueous vehicles; antimicrobial agents; isotonic agents; buffer agents; antioxidants; suspending/dispersing agents; chelating agents; diluents; adjuvants; excipients or non-toxic auxiliary substances; and other ingredients known in the art or various combinations thereof. In some embodiments, the excipients include diluents, fillers, binders, wetting agents, absorption enhancers, surfactants, lubricants, and/or stabilizers.

Different types of excipients may be selected according to different types of compositions. In some preferred embodiments, the aqueous cosmetic compositions may be selected from excipients such as water, ethanol, humectants, surfactants, softeners, and adhesives. In some preferred embodiments, the optional excipients for tablet pharmaceutical compositions or health-care product compositions include calcium bicarbonate, calcium phosphate, various types of sugars and starches, and cellulose derivatives.

### Uses

According to one embodiment of the present application, provided is use of the polypeptide of the present application in the preparation of an anti-UV, antioxidant, anti-inflammatory, or anti-aging product.

According to one embodiment of the present application, provided is use of a polypeptide having an amino acid sequence of TKAAA in the preparation of an anti-UV, antioxidant, anti-inflammatory, or skin anti-aging product.

Different types of products may be selected according to different purposes. In some embodiments, the composition includes cosmetics, health-care products, food, or pharmaceuticals. In some preferred embodiments, the product for alleviating UV damage, oxidation, inflammation, or aging of skin is a cosmetic.

Suitable dosage forms are well known to those skilled in the art. In some embodiments, the dosage form of the product includes injections, lyophilized powder injections, tablets, granules, aqueous solutions, oil formulations, creams, gels, emulsions, powders, or aerosols.

Different types of dosage forms may be selected according to different types of products. In some preferred embodiments, the optional dosage forms for the cosmetics include but are not limited to aqueous solutions, oil formulations, gels, creams, emulsions, powders, or aerosols. In some preferred embodiments, the optional dosage forms for the pharmaceuticals or health-care products include but are not limited to injections, lyophilized powder injections, tablets, or granules.

According to one embodiment of the present application, provided is use of the polypeptide of the present application and the composition of the present application in alleviating or treating UV damage, oxidation, inflammation, or aging.

In some embodiments, the product, the polypeptide, and the composition may be used to alleviate UV damage, oxidation, inflammation, or aging in any part of the body. In some preferred embodiments, the product, the polypeptide, and the composition are used to alleviate UV damage, oxidation, inflammation, or aging of skin.

According to one embodiment of the present application, provided is use of the polypeptide having an amino acid sequence of TKAAA in alleviating or treating UV damage, oxidation, inflammation, or skin aging.

In some embodiments, the polypeptide may be used to alleviate UV damage, oxidation, inflammation, or aging in any part of the body. In some preferred embodiments, the polypeptide may be used to alleviate UV damage, oxidation, inflammation, or aging of skin.

### Methods

According to one embodiment of the present application, provided is a method for alleviating or treating UV damage, oxidation, inflammation, or aging. The method includes administering to a subject in need thereof the polypeptide of the present application, the composition of the present application, and/or the polypeptide having an amino acid sequence of TKAAA.

In some embodiments, the product, the polypeptide, and the composition may be used to alleviate UV damage, oxidation, inflammation, or aging in any part of the body. In some preferred embodiments, the product, the polypeptide, and the composition are used to alleviate UV damage, oxidation, inflammation, or aging of skin.

In the method of the present application, the administration dosage of the polypeptide or the composition may depend upon several factors, including the severity and responsiveness of symptoms, the route of administration, time of therapy (several days to several months to several years), and the time to symptom improvement. Those skilled in the art may adjust the dosage regimen according to the specific situation of the patient, to provide a therapeutic response. For example, a single administration may be performed, several divided doses may be administered within a predetermined period of time, or the dose may be reduced or increased as indicated by the treatment situation. The specification of the dosage is determined by the unique characteristics of active compounds and the specific therapeutic effect to be achieved. The dosage value may vary with the type and severity of the condition to be alleviated. For any particular subject, the specific dosage regimen may be adjusted over time according to individual needs and the professional judgment of the therapeutic clinician.

The various embodiments and preferences described to the present application above may be combined with each other (as long as they are not inherently contradictory and are applicable to the uses of the present application), and the various embodiments formed by such combinations are considered as a part of the present application.

### Examples

The exemplary embodiments of the present application will be illustrated with reference to the accompanying drawings below, including various details of the embodiments of the present application to facilitate understanding. It should be understood that these exemplary embodiments are merely exemplary, but are not intended to limit the scope of protection of the present application. The scope of protection of the present application is merely defined by the claims. Therefore, those skilled in the art should recognize that various changes and modifications may be made to the embodiments described herein without departing from the scope of the present application. Similarly, the descriptions of well-known functions and structures are omitted in the following description for clarity and conciseness.

Unless otherwise specified, the reagents and instruments used in the following examples are conventional products that may be commercially obtainable. Unless otherwise specified, experiments are carried out under conventional conditions or under conditions recommended by the manufacturer.

In the present application, a mature human keratinocyte photodamage model is utilized to screen polypeptide structures with anti-aging efficacy. This model may effectively reflect whether the polypeptide has the efficacies of resisting UV damage, promoting cell proliferation, preventing oxidative damage, and resisting inflammatory injury, etc. Therefore, the human keratinocyte photodamage model may comprehensively evaluate the anti-aging efficacy of polypeptide drugs and their application potentials in cosmetics, health-care products, etc.

### Example 1: Cytotoxicity test of the polypeptides on keratinocytes

### 1.1 Experimental materials and instruments

Polypeptide LA-19 (having a sequence as shown in SEQ ID NO: 1), having a concentration of 1 pM-10 µM;
polypeptide TM-19 (having a sequence as shown in SEQ ID NO: 2), having a concentration of 1 pM-10 µM;
polypeptide P3 (having a sequence as shown in SEQ ID NO: 3), having a concentration of 1 nM-100 µM;
polypeptide P4 (having a sequence as shown in SEQ ID NO: 4), having a concentration of 1 nM-100 µM;
polypeptide P5 (having a sequence as shown in SEQ ID NO: 5), having a concentration of 1 nM-100 µM;
polypeptide P6 (having a sequence as shown in SEQ ID NO: 6), having an N-terminal modified by acetylation and a concentration of 1 nM-100 µM (the acetylation method: Fmoc is removed after coupling is complete, and the polypeptide is acetylated by 0.5 ml of acetic anhydride);
polypeptide P7 (having a sequence as shown in SEQ ID NO: 7), having a C-terminal modified by amidation and a concentration of 1 nM-100 µM (the amidation method: the polypeptide will automatically be amidated by using a Rink resin);
polypeptide P8 (having a sequence as shown in SEQ ID NO: 8), having a concentration of 1 nM-100 µM;
polypeptide P9 (having a sequence as shown in SEQ ID NO: 9), having an N-terminal modified by acetylation and having a concentration of 1 nM-100 µM;
polypeptide P10 (having a sequence as shown in SEQ ID NO: 10), having a concentration of 1 nM-100 µM;
polypeptide Acein (having a sequence of TKAAA) and having a concentration of 1 nM-100 µM;
cisplatin injection (Jiangsu Hansoh Pharmaceutical Group Co., Ltd.) as a positive control, having a concentration of 20 µg/mL.

Human keratinocytes (HaCaT cells, Nanjing Saihongrui Biotechnology Co., Ltd.).

Cell culture conditions: constant temperature and humidity incubator (37°C, 95% air, 5% carbon dioxide), 90% dulbecco's modified eagle medium (DMEM) high-glucose medium + 10% fetal bovine serum (Nanjing BioChannel Biotechnology Co., Ltd).

Trypsin-ethylene diamine tetraacetic acid (EDTA) solution (Nanjing BioChannel Biotechnology Co., Ltd); PBS solution (containing KH₂PO₄, Na₂HPO₄, NaCl, KCl, Nanjing Chemical Reagent Co., Ltd.); CCK-8 cell viability assay kit (Nanjing Enogene Biotech. Co., Ltd.).

96-well plate (SHANGHAI JING' AN BIOTECHNOLOGY CO., LTD); UPT ultrapure water system (Sichuan Ulupure Ultrapure Technology Co., Ltd.); microplate reader (Thermo Fisher Scientific (Shanghai) Instruments Co., Ltd.); analytical balance (Sartorius Scientific Instruments Co., Ltd.); low-speed centrifuge (Changsha Xiangzhi Centrifuge Instrument Co., Ltd.).

### 1.2 Experimental method

A cell culture flask was taken out from the cell incubator, observed under a microscope, and the cells were in the logarithmic growth phase. In a clean bench, the medium was discarded, the cells were rinsed with 2 mL of phosphate buffer saline (PBS) and discarded; after repeating the above process once, 2 mL of 0.25% trypsin was added for digestion. The digestion was terminated by adding a corresponding volume of serum-containing medium, and the cells were transferred to a 15 mL conical centrifuge tube and centrifuged at 1000 rpm for 5 minutes. The serum-containing medium was resuspended in a resuspension volume of 2 mL. The cells were counted and calculated. The cells were then diluted, and plated; then the diluted cell suspension was taken, then the 96-well plate was paved with 10⁴ cells/well, and the edge wells were filled with PBS. The well plate was taken out from the cell incubator, the medium was discarded, the well-plate of the administration group was added with the corresponding drugs and placed into the cell incubator for incubation for 48 hours. On the day of detection, the 96-well plate was taken out from the cell incubator, and added with 10 µL of a CCK-8 reagent per well in the clean bench, and then quickly placed into the cell incubator for 60 minutes, and the absorbance was measured with a microplate reader and the absorbance was measured at OD 450 nm. The average reading of the blank group was taken, and the numerical value of each group was divided by the average value to obtain the change in cell viability. Results are expressed as Mean ± SEM, compared with the blank group, *P<0.05 denotes a significant difference, **P<0.01, ***P<0.001, and ****P<0.0001 denote very significant differences.

### 1.3 Experimental results

In Table 1 and FIG. 1, the cells were not treated by the drug in the blank group, and cells in the positive control group were treated by 20 µg/mL of cisplatin. The results show that as compared to the blank group, in the polypeptide administration group, the polypeptides LA-19, P3, P4, P5, P6, P7, P8, P9, P10, and Acein do not significantly inhibit cell growth and may promote cell proliferation at certain concentrations. Among them, the polypeptide LA-19 has no obvious toxicity to keratinocytes and may promote cell proliferation, and its activity changes obviously with concentration. The polypeptide TM-19 may slightly inhibit cell growth at low concentrations but promote cell proliferation at high concentrations, and has a skin-repairing efficacy, and its activity changes obviously with concentration. In addition, compared with polypeptide Acein, polypeptides LA-19, TM-19, P7, and P9 may promote keratinocyte growth more significantly, especially LA-19, indicating that these polypeptides have a better skin-repairing efficacy. The above results verify that the eleven polypeptides are safe for human skin, and the experimental results are statistically significant.

**Table 1 Cytotoxicity assay of the polypeptides on keratinocytes**

| Group | Concentration of Administration | Cell Viability (%) |
|---|---|---|
| Control group | Blank group | 100.00±2.55 |
| | Positive control group | 26.36±0.61**** |
| LA-19 | 1 pM | 105.54±3.05 |
| | 10 pM | 110.27±1.65** |
| | 100 pM | 108.55±9.42** |
| | 1 nM | 115.51±7.42*** |
| | 10 nM | 119.18±10.57**** |
| | 100 nM | 117.14±10.03*** |
| | 1 µM | 118.65±9.32** |
| | 10 µM | 118.67±10.64**** |
| TM-19 | 1 pM | 81.97±4.23**** |
| | 10 pM | 90.31±7.57 |
| | 100 pM | 96.51±3.85 |
| | 1 nM | 102.51±9.07 |
| | 10 nM | 109.04±6.30 |
| | 100 nM | 112.71±5.15** |
| | 1 µM | 110.47±8.92** |
| | 10 µM | 116.29±2.98*** |
| P3 | 1 nM | 100.00±4.43 |
| | 10 nM | 104.23±7.30 |
| | 100 nM | 102.80±3.65 |
| | 1 µM | 100.74±3.63 |
| | 10 µM | 104.80±3.48 |
| | 100 µM | 102.71±4.61 |
| P4 | 1 nM | 100.00±5.11* |
| | 10 nM | 106.66±4.24*** |
| | 100 nM | 110.69±7.48 |
| | 1 µM | 105.69±4.44 |
| | 10 µM | 96.30±5.66 |
| | 100 µM | 99.72±6.12 |
| P5 | 1 nM | 103.68±4.60 |
| | 10 nM | 106.16±3.73** |
| | 100 nM | 105.24±3.81* |
| | 1 µM | 103.03±2.79 |
| | 10 µM | 99.72±2.93 |
| | 100 µM | 102.24±3.20 |
| P6 | 1 nM | 102.71±10.49 |
| | 10 nM | 104.78±8.23 |
| | 100 nM | 108.48±9.36 |
| | 1 µM | 108.86±7.39 |
| | 10 µM | 116.82±12.81** |
| | 100 µM | 101.94±6.67 |
| P7 | 1 nM | 100.41±4.00 |
| | 10 nM | 111.98±6.41** |
| | 100 nM | 107.14±5.98* |
| | 1 µM | 104.86±2.59 |
| | 10 µM | 112.41±2.65**** |
| | 100 µM | 108.72±4.95** |
| P8 | 1 nM | 99.66±7.91 |
| | 10 nM | 105.33±6.70 |
| | 100 nM | 106.68±8.00 |
| | 1 µM | 101.54±6.28 |
| | 10 µM | 100.17±7.20 |
| | 100 µM | 97.83±6.72 |
| P9 | 1 nM | 100.12±3.29 |
| | 10 nM | 99.15±8.60 |
| | 100 nM | 103.11±7.57 |
| | 1 µM | 108.53±1.17* |
| | 10 µM | 111.66±2.27*** |
| | 100 µM | 103.66±2.42 |
| P10 | 1 nM | 103.33±1.69 |
| | 10 nM | 107.63±2.77* |
| | 100 nM | 101.40±4.69 |
| | 1 µM | 90.59±3.51 |
| | 10 µM | 94.43±9.16 |
| | 100 µM | 92.18±5.72 |
| Acein | 1 nM | 104.33±2.75 |
| | 10 nM | 105.31±4.68 |
| | 100 nM | 102.38±4.30 |
| | 1 µM | 101.03±4.45 |
| | 10 µM | 101.25±4.43 |
| | 100 µM | 110.91±5.35** |

| | | |
|---|---|---|
| Note: the blank group is a serum-free medium without drugs, the positive control group is a 20 µg/mL cisplatin injection. The above results are expressed as Mean ± SEM; compared with the blank group, *P<0.05 denotes a significant difference, **P<0.01, ***P<0.001, and ****P<0.0001 denote very significant differences. | | |

### Example 2 Anti-UV activity test of the polypeptides on keratinocytes

### 2.1 Experimental materials and instruments

UVB lamp tube (GERMANY PULINSI INTERNATIONAL GROUP LIMITED); UV irradiance meter (Shenzhen Sanpo Instrument Co., Ltd.); retinol (Energy Chemical, Shanghai). Other experimental materials and instruments are the same as those in Example 1.

### 2.2 Experimental method

A cell culture flask was taken out from the cell incubator, observed under a microscope, and the cells were in the logarithmic growth phase. In a clean bench, the medium was discarded, the cells were rinsed with 2 mL of PBS and discarded; after repeating the above process once, 2 mL of 0.25% trypsin was added for digestion. Terminate the digestion using the corresponding volume contains the blood serum medium, and the cells were transferred to a 15 mL conical centrifuge tube and centrifuged at 1000 rpm for 5 minutes. The serum-containing medium was resuspended in a resuspension volume of 2 mL. The cells were counted and calculated. The cells were then diluted, and plated; then the diluted cell suspension was taken, then the 96-well plate was paved with 10⁴ cells/well, and the edge wells were filled with PBS. The 96-well plate was taken out from the cell incubator, the medium was discarded, the well-plate of the administration group was added with the corresponding drugs and placed into the cell incubator for incubation for 48 hours. After 24 hours of incubation, cell UV modeling was performed. The UVB lamp tube was turned on, and the radiation intensity was measured with a UV irradiance meter. When the radiation intensity reached 500 µW/cm², the uncovered 96-well plate was placed and irradiated for 1 minute such that the radiation dose was up to 30 mJ/cm². At the end of the radiation, the 96-well plate was covered and placed into the cell incubator. On the day of detection, the 96-well plate was taken out from the cell incubator, and added with 10 µL of a CCK-8 reagent per well in the clean bench, and then quickly placed into the cell incubator for 60 minutes, and the absorbance was measured with a microplate reader and the absorbance was measured at OD 450 nm. The average reading of the blank group was taken, and the numerical value of each group was divided by the average value to obtain the change in cell viability. Results are expressed as Mean ± SEM, compared with the blank group, in the model group, ####P<0.0001 denotes a significant difference; compared with the model group, in the administration group, *P<0.05 denotes a significant difference, **P<0.01, ***P<0.001, and ****P<0.0001 denote very significant differences.

### 2.3 Experimental results

In Table 2 and FIG. 2, neither UV irradiation nor drug treatment was performed in the blank group; the model group was subjected to UV irradiation but no drug treatment, and the positive control group was subjected to UV irradiation and 1 µM of retinol treatment. The results show that compared with the blank group, the model group has a significant difference, indicating the successful establishment of the UV damage model; compared with the model group, in the administration group, polypeptides LA-19, TM-19, P3, P4, P5, P6, P7, P8, P9, P10, and Acein all have certain anti-UV damage ability, slowing down UV irradiation-induced inhibition of cell growth, and their anti-UV damage activity shows obvious changes with concentration; moreover, at partial concentrations, the activity of damaged keratinocytes is restored to the level of the blank group. Compared with the positive control group, in the administration group, the anti-UV activity produced by the polypeptides at partial concentrations is higher than that of the positive drug 1 µM retinol. In addition, compared with polypeptide Acein, the polypeptides LA-19, TM-19, P6, P7, P8, P9, and P10 all may slow down the UV irradiation-induced inhibition of cell growth more significantly, and even made the activity of damaged keratinocytes higher than that of the cells in the blank group at partial concentrations, indicating that these polypeptides have better anti-UV damage activity.

UV radiation is a major cause of cellular DNA damage and may lead to cellular senescence (Stem Cells International, 2016, 2016: e7370642. DOI:10.1155/2016/7370642.). By alleviating the UV-induced damage, cellular senescence, especially skin cell senescence, may be effectively improved. Therefore, the above results show that the polypeptides provided in the present application may effectively improve the UV-induced skin damage and may be used to improve photooxidation-induced skin aging. The experimental results are statistically significant.

**Table 2 Anti-UV activity assay of the polypeptides on keratinocytes**

| Group | Concentration of Administration | Cell Viability (%) |
|---|---|---|
| Control group | Blank group | 100.00±2.76 |
| | Model group | 80.09±2.57#### |
| | Positive control group | 92.98±5.98** |
| LA-19 | 1 nM | 92.60+6.64** |
| | 10 nM | 89.89±2.52* |
| | 100 nM | 93.38±6.38** |
| | 1 µM | 95.75±4.73*** |
| | 10 µM | 102.56±8.45**** |
| | 100 µM | 91.12±5.60* |
| TM-19 | 1 nM | 85.23±3.67 |
| | 10 nM | 85.75±7.31 |
| | 100 nM | 86.31±5.80 |
| | 1 µM | 96.27±7.85** |
| | 10 µM | 94.98±6.56** |
| | 100 µM | 92.66±10.78** |
| P3 | 1 µM | 85.41±2.41 |
| | 10 µM | 87.55±2.05* |
| | 100 µM | 93.00±1.64**** |
| P4 | 1 µM | 86.81±1.59* |
| | 10 µM | 87.43±0.46* |
| | 100 µM | 87.21±2.24* |
| P5 | 1 µM | 89.49±2.96** |
| | 10 µM | 92.26±3.26**** |
| | 100 µM | 94.23±3.86**** |
| P6 | 1 µM | 110.86±3.03**** |
| | 10 µM | 108.53±2.05**** |
| | 100 µM | 105.99±2.34**** |
| P7 | 1 µM | 112.02±1.64**** |
| | 10 µM | 111.60±2.38**** |
| | 100 µM | 102.17±1.30**** |
| P8 | 1 µM | 110.01±4.10**** |
| | 10 µM | 108.94±1.16**** |
| | 100 µM | 110.37±3.76**** |
| P9 | 1 µM | 125.70±4.13**** |
| | 10 µM | 118.92±3.96**** |
| | 100 µM | 118.01±2.49**** |
| P10 | 1 µM | 129.42±4.21**** |
| | 10 µM | 127.52±4.15**** |
| | 100 µM | 116.31±1.76**** |
| Acein | 1 µM | 92.86±5.17*** |
| | 10 µM | 88.91±2.34* |
| | 100 µM | 96.04±4.67**** |

| | | |
|---|---|---|
| Note: the blank group is a serum-free medium without drugs and not subjected to UV modeling, the model group is a serum-free medium without drugs, and the positive control group is 1 µM retinol. The above results are expressed as Mean ± SEM, compared with the blank group, in model group, ####P<0.0001 denotes a very significant difference; compared with the model group, in the administration group, *P<0.05 denotes a significant difference, **P<0.01, ***P<0.001, and ****P<0.0001 denote very significant differences. | | |

### Example 3 Antioxidant activity test of the polypeptides on keratinocytes

### 3.1 Experimental materials and instruments

Superoxide dismutase (SOD) assay kit (Nanjing Jiancheng Bioengineering Research Institute Co., Ltd.); catalase (CAT) assay kit (Nanjing Jiancheng Bioengineering Research Institute Co., Ltd.); cell malondialdehyde (MDA) assay kit (Nanjing Jiancheng Bioengineering Research Institute Co., Ltd.); and 6-well plate (Wuxi NEST Biotechnology Co., Ltd.). Other experimental materials and instruments are the same as those in Examples 1 and 2.

### 3.2 Experimental method

A cell culture flask was taken out from the cell incubator, observed under a microscope, and the cells were in the logarithmic growth phase. In a clean bench, the medium was discarded, the cells were rinsed with 2 mL of PBS and discarded; after repeating the above process once, 2 mL of 0.25% trypsin was added for digestion. Terminate the digestion using the corresponding volume contains the blood serum medium, and the cells were transferred to a 15 mL conical centrifuge tube and centrifuged at 1000 rpm for 5 minutes. The serum-containing medium was resuspended in a resuspension volume of 2 mL. The cells were counted and calculated. The cells were then diluted, and plated; then the diluted cell suspension was taken, then the 6-well plate was paved with 3x10⁵ cells/well. The 6-well plate was taken out from the cell incubator, the medium was discarded, the well-plate of the administration group was added with the corresponding drugs and placed into the cell incubator for incubation for 48 hours. After 24 hours of incubation, cell UV modeling was performed. The UVB lamp tube was turned on, and the radiation intensity was measured with a UV irradiance meter. When the radiation intensity reached 500 µW/cm², the uncovered 6-well plate was placed and irradiated for 1 minute such that the radiation dose was up to 30 mJ/cm². At the end of the radiation, the 6-well plate was covered and placed into the cell incubator. On the day of detection, the 6-well plate was taken out from the cell incubator, the cells were collected in the clean bench and lysed to obtain a cellular content sample, then operated according to the instructions of each assay kit, and finally the absorbance was measured with a microplate reader to calculate the antioxidant-related activity values. The results are expressed as Mean ± SEM, compared with the blank group, in model group, #P<0.05 denotes a significant difference, ####P<0.0001 denotes a very significant difference; compared with the model group, in the administration group, *P<0.05 denotes a significant difference, **P<0.01, ***P<0.001, and ****P<0.0001 denote very significant differences.

### 3.3 Experimental results

In Table 3 and FIGs. 3-5, neither UV irradiation nor drug treatment was performed in the blank group; the model group was subjected to UV irradiation but no drug treatment, and the positive control group was subjected to UV irradiation and 1 µM of retinol treatment. The results show that compared with the blank group, the model group has a significant difference, indicating the successful establishment of the UV-induced oxidative damage model; compared with the model group, in the administration group, the polypeptides LA-19, TM-19, P3, P4, P5, P6, P7, P8, P9, P10, and Acein all have certain antioxidant activity, and may relieve the UV-induced cell oxidative damage. Compared with the positive control group, in the administration group, the antioxidant activity produced by the polypeptides at partial concentrations was higher than that of the positive drug 1 µM retinol. In addition, compared with polypeptide Acein, the polypeptides P6, P7, P8, P9, and P10 all may improve the antioxidant capacity of cells more significantly, indicating that these polypeptides have a better antioxidant efficacy.

Oxidative stress plays an important role in aging, especially skin aging (Antioxidants, 2022, 11(6): 1121. DOI:10.3390/antiox11061121.). In the aging process, the occurrence of oxidative stress is mainly attributed to the increase of reactive oxygen species and the decrease in the levels of enzymatic and non-enzymatic protective agents. Alleviating the damage caused by oxidative stress is beneficial to slowing down the aging process. Therefore, the above results indicate that the polypeptides provided in the present application may effectively improve cellular oxidative damage and may be used to improve the oxidative stress-induced aging, especially skin aging. The experimental results are statistically significant.

**Table 3 Antioxidant activity test of the polypeptides on keratinocytes**

| Group | Concentration of Administration | SOD Activity (U/mgprot) | CAT Activity (U/mgprot) | MDA Activity (U/mgprot) |
|---|---|---|---|---|
| Control | Blank group | 96.406±1.655 | 2.683±0.211 | 0.413±0.075 |
| | Model group | 81.925±2.524#### | 2.288±0.119# | 1.288±0.050#### |
| | Positive control group | 89.882+0.937**** | 2.977+0.060*** | 0.729+0.017**** |
| LA-19 | 1 µM | 83.266+0.899 | 1.879+0.155 | 0.619+0.040**** |
| | 10 µM | 87.238±1.709**** | 2.238±0.189 | 0.796±0.100**** |
| | 100 µM | 96.252±1.222**** | 2.926±0.354** | 0.707±0.050**** |
| TM-19 | 1 µM | 83.453±0.979 | 1.990±0.127 | 0.605±0.045**** |
| | 10 µM | 84.073±0.895 | 2.778±0.317* | 0.676±0.070**** |
| | 100 µM | 82.772±0.993 | 2.419±0.238 | 0.629±0.101**** |
| P3 | 1 µM | 92.369±0.576**** | 2.781±0.051**** | 1.018±0.083*** |
| | 10 µM | 93.142±1.058**** | 2.872±0.183**** | 1.007±0.028*** |
| | 100 µM | 87.552±1.060*** | 3.224±0.109**** | 0.938±0.067**** |
| P4 | 1 µM | 109.826±0.390**** | 2.689±0.070** | 1.017±0.048**** |
| | 10 µM | 98.207±1.152**** | 2.746±0.042*** | 1.055±0.046*** |
| | 100 µM | 98.422±0.358**** | 3.370±0.236**** | 0.989±0.057**** |
| P5 | 1 µM | 99.523±0.858**** | 2.842±0.104**** | 1.158±0.035 |
| | 10 µM | 85.166±1.044* | 3.323±0.049**** | 1.230±0.086 |
| | 100 µM | 85.809±0.332** | 3.287±0.120**** | 1.236±0.043 |
| P6 | 1 µM | 101.823±0.926**** | 3.395±0.084**** | 1.057±0.090** |
| | 10 µM | 95.147±0.370**** | 3.327±0.069**** | 1.067±0.032** |
| | 100 µM | 85.499±0.392* | 2.628±0.024**** | 1.138±0.099 |
| P7 | 1 µM | 99.309±0.137**** | 3.342±0.118**** | 0.920±0.031**** |
| | 10 µM | 96.129±1.205**** | 3.120±0.118**** | 1.038±0.026**** |
| | 100 µM | 84.359±2.235 | 2.656±0.081*** | 1.098±0.010*** |
| P8 | 1 µM | 96.851±1.066**** | 3.268±0.097**** | 1.006±0.020**** |
| | 10 µM | 98.006±0.266**** | 3.346±0.079**** | 1.005±0.053**** |
| | 100 µM | 98.372±0.922**** | 3.802±0.196**** | 1.034±0.047**** |
| P9 | 1 µM | 91.724±0.207**** | 3.665±0.215**** | 0.780±0.034**** |
| | 10 µM | 94.461±0.793**** | 3.438±0.117**** | 0.998±0.017**** |
| | 100 µM | 87.008±0.553*** | 2.713±0.093*** | 1.039±0.006**** |
| P10 | 1 µM | 100.822±0.627**** | 3.332±0.071**** | 0.793±0.012**** |
| | 10 µM | 97.685±0.922**** | 3.730±0.074**** | 0.788±0.033**** |
| | 100 µM | 83.280±0.554 | 2.476±0.065* | 0.993±0.045**** |
| | 1 µM | 88.875±2.654**** | 2.152±0.068 | 1.209±0.072 |
| Acein | 10 µM | 99.499±2.912**** | 2.180±0.021 | 1.250±0.024 |
| | 100 µM | 98.908±2.677**** | 2.994±0.104**** | 1.102±0.051** |

| | | | | |
|---|---|---|---|---|
| Note: the blank group is a serum-free medium without drugs and not subjected to UV modeling, the model group is a serum-free medium without drugs, the positive control group is 1 µM retinol. The above results are expressed as Mean ± SEM; compared with the blank group, in the model group, #P<0.05 denotes a significant difference, ####P<0.0001 denotes a very significant difference; compared with the model group, in the administration group, *P<0.05 denotes a significant difference, **P<0.01, ***P<0.001, and ****P<0.0001 denote very significant differences. | | | | |

### Example 4: Anti-inflammatory activity test of the polypeptides on keratinocytes

### 4.1 Experimental materials and instruments

Interleukin-6 ELISA assay kit (Shanghai Beyotime Biotechnology Co., Ltd.); tumor necrosis factor α ELISA assay kit (Shanghai Beyotime Biotechnology Co., Ltd.); 6-well plate (Wuxi NEST Biotechnology Co., Ltd.); and palmitoyl tetrapeptide-7 (Shenzhen Jianyuan Pharmaceutical Technology Co., Ltd.). Other experimental materials and instruments are the same as those in Examples 1, 2, and 3.

### 4.2 Experimental method

A cell culture flask was taken out from the cell incubator, observed under a microscope, and the cells were in the logarithmic growth phase. In a clean bench, the medium was discarded, the cells were rinsed with 2 mL of PBS and discarded; after repeating the above process once, 2 mL of 0.25% trypsin was added for digestion. Terminate the digestion using the corresponding volume contains the blood serum medium, and the cells were transferred to a 15 mL conical centrifuge tube and centrifuged at 1000 rpm for 5 minutes. The serum-containing medium was resuspended in a resuspension volume of 2 mL. The cells were counted and calculated. The cells were then diluted, and plated; then the diluted cell suspension was taken, then the 6-well plate was paved with 3x10⁵ cells/well. The 6-well plate was taken out from the cell incubator, the medium was discarded, the well-plate of the administration group was added with the corresponding drugs and placed into the cell incubator for incubation for 48 hours. After 24 hours of incubation, cell UV modeling was performed. The UVB lamp tube was turned on, and the radiation intensity was measured with a UV irradiance meter. When the radiation intensity reached 500 µW/cm², the uncovered 6-well plate was placed and irradiated for 1 minute such that the radiation dose was up to 30 mJ/cm². At the end of the radiation, the 6-well plate was covered and placed into the cell incubator. On the day of detection, the 6-well plate was taken out from the cell incubator, the cells were collected in the clean bench and centrifuged, and the resulting supernatant (cell culture medium) was collected for analysis, then operated according to the instructions of each ELISA assay kit, and finally the absorbance was measured with a microplate reader to calculate the antioxidant-related activity values. The results are expressed as Mean ± SEM, compared with the blank group, in model group, ###P<0.001 denotes a significant difference, ####P<0.0001 denotes a very significant difference; compared with the model group, in the administration group, *P<0.05 denotes a significant difference, **P<0.01, ***P<0.001, and ****P<0.0001 denote very significant differences.

### 4.3 Experimental results

In Table 4 and FIGs. 6-7, neither UV irradiation nor drug treatment was performed in the blank group; the model group was subjected to UV irradiation but no drug treatment, and the positive control group was subjected to UV irradiation and 10 µM of palmitoyl tetrapeptide-7. The results show that compared with the blank group, the model group has a significant difference, indicating the successful establishment of the UV-induced cell inflammation model; compared with the model group, in the administration group, the polypeptides LA-19, TM-19, P3, P4, P5, P6, P7, P8, P9, P10, and Acein all have certain anti-inflammatory ability, and may relieve the degree of UV-induced cell inflammation. Compared with the positive control group, in the administration group, the anti-inflammatory activity produced by the polypeptides at partial concentrations was higher than that of the positive drug, i.e., 10 µM of palmitoyl tetrapeptide-7. In addition, compared with polypeptide Acein, the polypeptides P6, P7, P8, P9, and P10 all may improve the antioxidant capacity of cells more significantly, indicating that these polypeptides have a better anti-inflammatory efficacy.

Studies have found that chronic inflammation may accelerate the aging process via reactive oxygen species-mediated telomere dysfunction and cellular senescence (Nature Communications, 2014, 5(1): 4172. DOI:10.1038/ncomms5172.). In addition, studies have also found that the UV radiation-induced inflammation may accelerate the skin aging process (Inflammation Research, 2022, 71(7): 817-831. DOI:10.1007/s00011-022-01598-8.). Therefore, the above results indicate that the polypeptides provided in the present application have good anti-inflammatory activity and may be used to improve inflammation-related aging, especially skin aging. The experimental results are statistically significant.

**Table 4 Anti-inflammatory activity test of the polypeptides on keratinocytes**

| Group | Concentration of Administration | IL-6 activity (pg/mgprot) | TNF-α activity (pg/mgprot) |
|---|---|---|---|
| Control | Blank group | 10.619±0.340 | 2.890±1.724 |
| | Model group | 18.622±0.512#### | 10.812±0.905### |
| | Positive control group | 13.901±1.356**** | 6.832±2.461* |
| LA-19 | 1 µM | 13.448±0.906**** | 5.267±0.472** |
| | 10 µM | 13.846±0.889**** | 8.042±0.997* |
| | 100 µM | 15.683±1.683* | 7.332±1.702 |
| TM-19 | 1 µM | 11.657±0.656**** | 8.148±0.231 * |
| | 10 µM | 11.756±1.167**** | 5.565±0.240* |
| | 100 µM | 14.087±0.791 *** | 7.119±1.413* |
| P3 | 1 µM | 13.327±0.405**** | 6.542±0.202* |
| | 10 µM | 13.516±0.164**** | 5.960±1.257* |
| | 100 µM | 12.423±1.359**** | 5.720±0.318** |
| P4 | 1 µM | 12.309±0.896*** | 6.474±0.790* |
| | 10 µM | 12.319±2.450**** | 6.291±0.372* |
| | 100 µM | 11.838±0.085*** | 4.610±0.360** |
| P5 | 1 µM | 12.642±1.711*** | 5.931±0.364** |
| | 10 µM | 13.226±0.891*** | 5.587±0.361 ** |
| | 100 µM | 12.820±1.105*** | 5.332±0.063** |
| P6 | 1 µM | 11.771±0.466**** | 8.535±0.523* |
| | 10 µM | 11.671±0.414**** | 9.907±0.215 |
| | 100 µM | 11.440±0.314**** | 9.747±0.352 |
| P7 | 1 µM | 11.904±0.664**** | 8.967±0.581* |
| | 10 µM | 11.835±0.310**** | 9.409±0.509 |
| | 100 µM | 11.829±0.333**** | 10.506±1.189 |
| P8 | 1 µM | 11.485±1.463**** | 8.619±0.675* |
| | 10 µM | 11.392±1.056**** | 8.989±0.252* |
| | 100 µM | 12.070±0.676**** | 8.408±0.423* |
| P9 | 1 µM | 11.300±1.461**** | 8.186±0.469* |
| | 10 µM | 10.702±0.748**** | 7.731±0.982* |
| | 100 µM | 10.478±0.783**** | 7.242±0.405** |
| P10 | 1 µM | 11.844±1.095**** | 7.807±0.439** |
| | 10 µM | 10.819±0.564**** | 7.812±0.500** |
| | 100 µM | 11.310±1.057**** | 8.118±0.916* |
| Acein | 1 µM | 20.356±0.479 | 6.129±0.582** |
| | 10 µM | 20.421±0.919 | 6.072±0.746** |
| | 100 µM | 17.586±1.310 | 5.540±0.284** |

| | | | |
|---|---|---|---|
| Note: the blank group is a serum-free medium without drugs and not subjected to UV modeling, the model group is a serum-free medium without drugs, the positive control group is 10 µM of palmitoyl tetrapeptide-7. The above results are expressed as Mean ± SEM; compared with the blank group, in the model group, ###P<0.001 and ####P<0.0001 denote very significant differences; compared with the model group, in the administration group, *P<0.05 denotes a significant difference, **P<0.01, ***P<0.001, and ****P<0.0001 denote very significant differences. | | | |

It needs to be stated that the above are merely preferred embodiments of the present application but are not construed as limiting the present application. For those skilled in the art, the present application may have various changes and modifications. Although detailed embodiments have been described, for the applicant or other skilled artisans, there probably exist or currently unforeseeable alternatives, amendments, variations, improvements, and substantive equivalents to the above embodiments. Therefore, the appended claims and claims that may be probably modified are intended to cover all such alternatives, amendments, variations, improvements, and substantial equivalents. It is important that as technology evolves, many of the elements described herein may be replaced by equivalent elements that emerge after the present application.

## Claims

1. A polypeptide, comprising at least one amino acid sequence having at least 80%, 90%, 95%, or 99% identity to an amino acid sequence as shown in any one of SEQ ID NOs: 1-10.

2. The polypeptide according to claim 1, wherein the polypeptide is the amino acid sequence as shown in any one of SEQ ID NOs: 1-10.

3. The polypeptide according to claim 1, wherein the polypeptide further has a terminal modification, wherein the terminal modification comprises acetylation and/or amidation.

4. A composition for relieving or treating ultraviolet damage, oxidation, inflammation, or aging, comprising:
a polypeptide having an amino acid sequence as shown in any one of SEQ ID NOs: 1-10; and
a pharmaceutically, nutritionally or physiologically acceptable excipient.

5. The composition according to claim 4, wherein the ultraviolet damage, oxidation, inflammation, or aging comprises ultraviolet damage, oxidation, inflammation, or aging of skin.

6. The composition according to claim 4, wherein the composition comprises a cosmetic composition, a health-care product composition, a food composition, or a pharmaceutical composition.

7. The composition according to claim 4, wherein the excipient comprises a diluent, a filler, a binder, a humectant, an absorption enhancer, a surfactant, a lubricant, and/or a stabilizer.

8. Use of the polypeptide according to any one of claims 1-3 in the preparation of an anti-ultraviolet, anti-oxidant, anti-inflammatory, or anti-aging product.

9. Use of a polypeptide having an amino acid sequence of TKAAA in the preparation of an anti-ultraviolet, antioxidant, anti-inflammatory, or skin anti-aging product.

10. The use according to claim 8 or 9, wherein the product comprises cosmetics, a health-care product, food, or a medicament.

11. Use of the polypeptide according to any one of claims 1-3, or the composition according to any one of claims 4-7 in relieving or treating ultraviolet damage, oxidation, inflammation, or aging.

12. The use according to claim 11, wherein the ultraviolet damage, oxidation, inflammation, or aging comprises ultraviolet damage, oxidation, inflammation, or aging of skin.

13. Use of a polypeptide having an amino acid sequence of TKAAA in relieving or treating ultraviolet damage, oxidation, inflammation, or skin aging.
